## Europäisches Patentamt
## European Patent Office
## Office européen des brevets

(19)

(11) Veröffentlichungsnummer : **0 180 785**
**B1**

(12)

# EUROPÄISCHE PATENTSCHRIFT

(45) Veröffentlichungstag der Patentschrift :
19.07.89

(21) Anmeldenummer : 85112661.5

(22) Anmeldetag : 07.10.85

(51) Int. Cl.⁴ : **A 61 K 37/64** // (A61K37/64, 31:445)

(54) **Arzneimittel, enthaltend eine Kombination von Nitrendipin mit Enalapril, und Verfahren zu seiner Herstellung.**

(30) Priorität : 17.10.84 DE 3437917

(43) Veröffentlichungstag der Anmeldung :
14.05.86 Patentblatt 86/20

(45) Bekanntmachung des Hinweises auf die Patenterteilung : 19.07.89 Patentblatt 89/29

(84) Benannte Vertragsstaaten :
AT BE CH DE FR GB IT LI NL SE

(56) Entgegenhaltungen :
CHEMICAL ABSTRACTS, Band 102, Nr. 1, 7. Januar
1985, Seite 35, Zusammenfassung 372x, Columbus,
Ohio, US; M.E. VINCENT et al.: "Hemodynamic and
humoral responses to enalapril and nifedipine in the
rat" & CLIN. EXP. HYPERTENS., PART A 1984, A6(8),
1485-97
CHEMICAL ABSTRACTS, Band 100, Nr. 23, 4. Juni
1984, Seite 40, Zusammenfassung 185552f, Columbus, Ohio, US; P:S. CHAN et al.: "Acute antihypertensive synergism of angiotensin-converting enzyme
inhibitors and duretics" & FED. PROC. FED. AM. SOC.
EXP. BIOL. 1984, 43(5), 1346-50

(73) Patentinhaber : BAYER AG

D-5090 Leverkusen 1 Bayerwerk (DE)

(72) Erfinder : Garthoff, Bernward, Dr.
Händelstrasse 22
D-4010 Hilden (DE)
Erfinder : Kazda, Stanislav, Dr.
Gellertweg 18
D-5600 Wuppertal 1 (DE)
Erfinder : Knorr, Andreas, Dr.
Pahlkestrasse 15
D-5600 Wuppertal 1 (DE)

Anmerkung : Innerhalb von neun Monaten nach der Bekanntmachung des Hinweises auf die Erteilung des
europäischen Patents im Europäischen Patentblatt kann jedermann beim Europäischen Patentamt gegen das erteilte
europäische Patent Einspruch einlegen. Der Einspruch ist schriftlich einzureichen und zu begründen. Er gilt erst als
eingelegt, wenn die Einspruchsgebühr entrichtet worden ist (Art. 99(1) Europäisches Patentübereinkommen).

**Beschreibung**

Die vorliegende Erfindung betrifft eine Kombination der Dihydropyridinverbindung Nitrendipin mit einer Verbindung, die die Bildung von Enzymen hemmt, welche die Umwandlung von Angiotensin I in Angiotensin II steuern, in Arzneimitteln, insbesondere in blutdrucksenkenden Arzneimitteln.

Verbindungen, die die Bildung von Enzymen hemmen, die Angiotensin I in Angiotensin II umwandeln, werden als ACE-Hemmer bezeichnet (Angiotensin converting enzyme). Diese Verbindungen sind als blutdrucksenkendes Mittel bekannt, da sie den Blutdruck dann senken, wenn der Bluthochdruck auf Angiotensin II zurückgeführt werden kann.

ACE-hemmende Verbindungen müssen bei ihrer therapeutischen Anwendung bei einer Vielzahl hypertensiver Patienten in relativ hohen Dosierungen eingesetzt werden, was zur Beeinträchtigung der Patienten durch Nebenwirkungen führen kann.

Die ACE-hemmende Verbindung ist N-(1-(S)-Ethoxycarbonyl-3-phenylpropyl)-L-alanyl-L-prolin (Enalapril) oder dessen Maleatsalz.

Die Herstellung dieser Verbindung ist in der EP-A-12 401 beschrieben.

Dihydropyridine sind als blutdrucksenkende Verbindungen bekannt, wobei diese Wirkung durch Hemmung des Calciumeinstroms in die glatte Muskelzelle des Blutgefäßes eintritt.

Als erfindungsgemäßes Dihydropyridin sei Nitrendipin genannt.

Die vorliegende Erfindung beruht auf der Feststellung, daß durch Kombination mit dem Dihydropyridin eine überraschende Wirkungsverstärkung des ACE-Hemmers eintritt und somit eine Verringerung der Dosis ermöglicht wird.

Bezogen auf 1 Gewichtsteil des ACE-Hemmers können 1-10 Gewichtsteile, bevorzugt 1-3 Gewichtsteile, insbesondere 3 Gewichtsteile des Dihydropyridins eingesetzt werden.

Die Kombination kann hergestellt werden, indem man die Einzelkomponenten in diese auflösenden inerten Lösungsmitteln auflöst und nach Abdampfen des Lösungsmittels die Kombination in üblicher Weise mit Hilfsstoffen vermischt.

Als inerte Lösungsmittel seien beispielhaft Alkohole, wie Ethanol, oder Glykole, wie Polyethylenglykol, genannt.

Wie bereits erwähnt, kann die erfindungsgemäße Kombination zur Bekämpfung von Erkrankungen, besonders von Kreislauferkrankungen, insbesondere zur Behandlung der Hypertonie, der Herzinsuffizienz und/oder der koronaren Herzerkrankung verwendet werden.

Die Wirkstoffkombination kann in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht-toxischer pharmazeutisch geeigneter Trägerstoffe oder Lösungsmittel. Hierbei soll die Kombination der therapeutisch wirksamen Verbindungen jeweils in einer Konzentration von etwa 0,5 bis 50 Gew.-% der Gesamtmischung vorhanden sein, d. h. in Mengen, die ausreichend sind, um den angegebenen Dosierungsspielraum zu erreichen.

Diese Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z. B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt :

Wasser, nicht-toxische organische Lösungsmittel, wie Paraffine (z. B. Erdölfraktionen), pflanzliche Öle (z. B. Erdnuß-/Sesam-Öl), Alkohole (z. B. Ethylalkohol, Glycerin), Glykole (z. B. Propylenglykol, Polyethylenglykol), feste Trägerstoffe, wie z. B. natürliche Gesteinsmehle (z. B. Kaoline, Tonerden, Talkum, Kreide), synthetische Gesteinsmehle (z. B. hochdisperse Kieselsäure, Silikate), Zucker (z. B. Rohr-, Milch- und Traubenzucker), Emulgiermittel (z. B. Polyoxyethylen-Fettsäure-Ester, Polyoxyethylen-Fettalkohol-Ether, Alkylsulfonate und Arylsulfonate), Dispergiermittel (z. B. Lignin, Sulfitablaugen, Methylcellulose, Stärke und Polyvinylpyrrolidon) und Gleitmittel (z. B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer mit den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Die antihypertensive Wirksamkeit der erfindungsgemäßen Kombination Enalapril und Nitrendipin nach oraler Gabe wurde an wachen Ratten bestimmt, bei denen durch Unterbindung der Hauptschlagader zwischen den beiden Nierenarterien ein Bluthochdruck erzeugt worden war. Zur Erfassung des Blutdruckes über 24 Stunden wurde die spezielle Versuchsanordnung nach Garthoff und Towart (J. Pharmacol. Methods 5, 275-278, 1981) angewandt.

Orale Gabe von Nitrendipin führt zu dosisabhängiger, aber nur zeitlich begrenzter Blutdrucksenkung. Enalapril verursacht zwar bei diesen Ratten eine anhaltende Blutdrucksenkung, aber durch gleichzeitige Gabe einer geringen Dosis Nitrendipin läßt sich die Wirkung wesentlich verstärken (s. Tabelle). Andererseits verlängert die zusätzliche Gabe von Enalapril zu Nitrendipin die Blutdrucksenkung über 9 Stunden nach Gabe hinaus. Dies läßt zu, daß bei verbesserter und verlängerter Wirkung die Einzelkomponenten verringert werden beziehungsweise die Einzeldosen halbiert werden können (siehe Tabelle).

Somit ist es vorteilhaft, die beim Menschen üblichen Dosierungen (i. e. 2,5-40 mg Enalapril/die bzw. 10-60 mg Nitrendipin/die) in der Kombination begrenzen zu können. Vorzugsweise werden bei oraler Applikation die beiden Stoffe in einer Kombination von 2,5-15 mg (Enalapril), bzw. 10-20 mg (Nitrendipin) pro die verabreicht.

Trotzdem kann es gegebenenfalls erforderlich sein, von den genannten Mengen abzuweichen, und zwar in Abhängigkeit vom Körpergewicht bzw. der Art des Applikationsweges, aber auch aufgrund des individuellen Verhaltens gegenüber der Kombination bzw. der Art der Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte Grenze überschritten werden muß. Im Falle der Applikation größerer Mengen kann es empfehlenswert sein, diese in mehreren Einzelgaben über den Tag zu verteilen.

Siehe Tabelle 1 Seite 4 ff.)

Tabelle 1 : Blutdruckwerte (systolisch obere Hälfte, diastolisch untere Hälfte) in mm Hg vor und nach oraler Gabe des Angiotensin-converting enzyme-Inhibitors Enalapril oder des Calciumantagonisten Nitrendipin sowie der Kombination beider Stoffe.
n = Anzahl der Versuche, angegeben sind die Mittelwerte mit Streuung (SEM)

|  |  | Minuten nach Applikation | | | | | | Stunden nach Applikation | | | |
|---|---|---|---|---|---|---|---|---|---|---|---|
|  | n | 0 | 12 | 24 | 36 | 48 | 60 | 2 h | 4 h | 6 h | 9 h |
| Enalapril, 1 mg/kg p.o. | 10 | 198+6 | 195+6 | 170+7 | 165+8 | 164+5 | 158+4 | 167+7 | 173+9 | 175+8 | 194+11 |
| Nitrendipin 3,15 mg/kg p.o. | 6 | 212+8 | 146+14 | 144+8 | 138+7 | 142+7 | 146+6 | 174+8 | 201+9 | 208+10 | 227+7 |
| Kombination von Enalapril (1 mg/ kg p.o.) und Nitrendipin (3,15 mg/kg p.o.) | 10 | 202+3 | 163+6 | 138+6 | 132+4 | 134+5 | 138+5 | 152+5 | 168+4 | 175+4 | 185+8 |
| Kombination von Enalapril (0,5 mg/kg p.o.) und Nitrendipin (1,6 mg/kg p.o.) | 9 | 221+8 | 162+9 | 142+9 | 146+10 | 149+11 | 150+10 | 165+4 | 180+7 | 184+4 | 192+12 |

EP 0 180 785 B1

Tabelle 1    (Fortsetzung)

| | | | | | | | | | | | |
|---|---|---|---|---|---|---|---|---|---|---|---|
| Enalapril 1 mg/kg p.o. | 10 | 133±3 | 133±3 | 118±6 | 111±6 | 113±5 | 105±5 | 111±7 | 112±6 | 111±6 | 121±6 |
| Nitrendipin 3,15 mg/kg p.o. | 6 | 142±2 | 110±7 | 87±6 | 88±5 | 92±4 | 97±5 | 118±6 | 134±6 | 137±4 | 146±4 |
| Kombination von Enalapril (1 mg/ kg p.o.) und Nitrendipin (3,15 mg/kg p.o.) | 10 | 141±3 | 121±4 | 95±5 | 87±3 | 92±4 | 94±5 | 104±5 | 117±7 | 116±7 | 123±5 |
| Kombination von Enalapril (0,5 mg/kg p.o.) und Nitrendipin (1,6) mg/kg p.o.) | 9 | 138±7 | 107±4 | 89±4 | 92±4 | 92±4 | 95±4 | 108±5 | 115±7 | 118±7 | 120±10 |

EP 0 180 785 B1

## Patentansprüche

1. Arzneimittel mit verstärkter Hemmwirkung auf Enzyme, die die Umwandlung von Angiotensin I in Angiotensin II steuern und somit den durch pathologisch erhöhte Konzentration von Hormonen des Renin-Angiotensin-Systems verursachten Blutdruck senken, enthaltend eine Kombination von 1 Gew.-Teil des ACE-Hemmers N-(1-(S)-Ethoxycarbonyl-3-phenylpropyl)-L-alanyl-L-prolin oder dessen Maleatsalz und 1-10 Gew.-Teil(en) Nitrendipin.

2. Arzneimittel gemäß Anspruch 1, dadurch gekennzeichnet, daß sie eine Kombination von 1 Gew.-Teil des ACE-Hemmers und 1-3 Gew.-Teile Nitrendipin enthalten.

3. Verfahren zur Herstellung des Arzneimittels mit verstärkter blutdrucksenkender Wirkung enthaltend eine Kombination von 1 Gew.-Teil des ACE-Hemmers N-(1-(S)-Ethoxycarbonyl-3-phenylpropyl)-L-alanyl-L-prolin oder dessen Maleatsalz und 1-10 Gew.-Teile Nitrendipin, dadurch gekennzeichnet, daß man den ACE-Hemmer und Nitrendipin in inerten organischen Lösungsmitteln auflöst und nach Abdampfen des Lösungsmittels die erhaltene feste Kombination gegebenenfalls unter Verwendung üblicher Hilfsstoffe in eine übliche Applikationsform überführt.

4. Verfahren zur Herstellung des Arzneimittels gemäß Anspruch 3, dadurch gekennzeichnet, daß man als Lösungsmittel Alkohole oder Glykole einsetzt.

## Claims

1. Medicaments having an enhanced inhibitory action on enzymes which control the conversion of angiotensin I into angiotensin II and thus reduce the blood pressure caused by pathologically increased concentrations of hormones of the renin-angiotensin system, containing a combination of 1 part by weight of the ACE inhibitor N-(1(S)-ethoxycarbonyl-3-phenylpropyl)-L-alanyl-L-proline or its maleate salt and 1-10 part (s) by weight of nitrendipine.

2. Medicaments according to Claim 1, characterized in that they contain a combination of 1 part by weight of the ACE inhibitor and 1-3 parts by weight of nitrendipine.

3. Process for the preparation of the medicament having an enhanced antihypertensive action and containing a combination of 1 part by weight of the ACE inhibitor N(1-(S)-ethoxycarbonyl-3-phenylpropyl)-L-alanyl-L-proline or its maleate salt and 1-10 parts by weight of nitrendipine, characterized in that the ACE inhibitor and nitrendipine are dissolved in inert organic solvents and, after the solvent has been evaporated off, the resulting solid combination is converted into a customary administration form, optionally with the use of customary auxiliaries.

4. Process for the preparation of the medicament according to Claim 3, characterized in that the solvents employed are alcohols or glycols.

## Revendications

1. Médicaments produisant une action inhibitrice renforcée sur les enzymes qui commandent la conversion de l'angiotensine I en angiotensine II et qui réduisent ainsi l'hypertension sanguine causée par une concentration pathologiquement accrue des hormones du système rénine-angiotensine, contenant une combinaison de 1 partie en poids de l'inhibiteur d'ACE (Angiotensin converting enzyme = enzyme de conversion de l'angiotensine) N-(1-(S)éthoxycarbonyl-3-phénylpropyl)-1-alanyl-L-proline ou de son sel maléate et de 1 à 10 parties en poids de nitrendipine.

2. Médicaments selon la revendication 1, caractérisés en ce qu'ils contiennent une combinaison de 1 partie en poids de l'inhibiteur d'ACE et de 1 à 3 parties en poids de nitrendipine.

3. Procédé de fabrication du médicament à action hypotensive renforcée contenant une combinaison de 1 partie en poids de l'inhibiteur d'ACE N-(1-(S)éthoxycarbonyl-3-phénylpropyl)-L-alanyl-L-proline ou de son sel maléate et de 1 à 10 parties en poids de nitrendipine, caractérisé en ce qu'on dissout l'inhibiteur d'ACE et la nitrendipine dans des solvants organiques inertes et, après évaporation du solvant, en ce qu'on transporte le composé solide ainsi obtenu dans une forme d'application courante, éventuellement en utilisant des produits auxiliaires usuels.

4. Procédé de fabrication du médicament selon la revendication 3, caractérisé en ce qu'on utilise des alcools ou des glycols comme solvant.